# EUROPEAN PATENT APPLICATION

(11) **EP 0 719 789 A1**
(43) Date of publication of application: **03.07.1996**
(21) Application number: 95120690.3
(22) Date of filing: 28.12.1995
(51) Int. Cl.: C07K 5/06

(54) **Process of producing alpha-L-aspartyldipeptide amide derivatives**

(30) Priority: 28.12.1994 JP 327094/94; 24.08.1995 JP 215940/95
(71) Applicant: Ajinomoto Co., Ltd., Tokyo (JP)
(72) Inventor: Hijiya, Toyoto, c/o Central Research Lab., Kawasaki-ku, Kawasaki-shi, Kanagawa-ken (JP); Takemoto,Tadashi, c/o Cetral Research Lab., Kawasaki-ku, Kawasaki-shi, Kanagawa-ken (JP); Nakamura, Ryoichiro c/o Central Research Lab., Kawasaki-ku, Kawasaki-shi, Kanagawa-ken (JP); Amino, Yusuke c/o Central Research Lab., Kawasaki-ku, Kawasaki-shi, Kanagawa-ken (JP); Sugiyami, Naoko c/o Central Research Lab., Kawasaki-ku, Kawasaki-shi, Kanagawa-ken (JP)
(74) Representative: Strehl Schübel-Hopf Groening & Partner

(57) **Abstract**

The object of the present invention is to provide a useful process of producing α-L-aspartyldipeptide amide derivative.

The amino group of a L-aspartic acid β-alkylester is protected with acetoacetate or β-diketone. The resulting compound is converted into a mixed anhydride using an alkyl chloroformate, condensed with amino acid amide derivatives, and then hydrolyzed under acidic conditions to effect deprotection of the amino protective group and conversion of ester moiety into a carboxylic acid, whereby the target α-L-aspartyldipeptide amide derivatives are obtained.

## Description

### FIELD OF THE INVENTION

This invention relates to a process of producing artificial sweetener of α-L-aspartyldipeptide amides.

### BACKGROUND OF THE INVENTION

α-L-aspartyl-D-amino acid-N-(S)-α-alkylbenzylamide included in the invention is a artificial sweetener disclosed in US Pat. No. 5,286,509. The patent discloses a process of producing the compound N-benzyloxycarbonyl-L-aspartic acid-β-benzylester, protecting the amino group and β-carboxyl group in L-aspartic acid and condensing the compound with D-amino acid-N-(S)-α-alkylbenzylamide using dicyclohexylcarbodiimide; then a benzyloxycarbonyl group and a benzyl group are removed by catalytic reduction to obtain the objective compound. This method requires the use of expensive dicyclohexylcarbodiimide in the industrial production. In addition, the objective compound may contain a small amount of dicyclohexylurea derived from the reaction of dicyclohexylcarbodiimide, resulting in inferior quality.

The patent states that N-benzyloxycarbonyl-L-aspartic acid anhydride or N-formyl-L-aspartic acid anhydride can be replaced with the compounds as mentioned in the previous method, however, no specific methods or conditions are disclosed; β-L-aspartyl-D-amino acid amide derivatives are produced as by-products other than the objective α-L-aspartyl-D-amino acid amino derivatives when the amino group of D-amino acid amide attacks the carbonyl carbon of the carboxyl group at β-position of aspartic acid; the β by-products need to be removed as well and reduce production yields of the target compound..

### OBJECTIVE OF THE INVENTION

The objective of the invention is to provide the economical process of producing α-L-aspartyldipeptide amide derivatives without using expensive dicyclohexylcarbodiimide ad without yielding β by-products.

### SUMMARY OF THE INVENTION

The method of obtaining the objective α-L-aspartyldipeptide amide derivatives was investigated: aspartic acid derivative of formula (1) was mixed with alkyl chloroformate to produce a mixed anhydride, and was mixed with the amino acid amide derivative of formula (2) to produce protected α-L-aspartyldipeptide amide derivative with an excellent yield, then the compound was hydrolyzed under acidic conditions to remove the amino protecting group of aspartic acid and to convert β-carboxylic acid ester group to the carboxylic acid group. 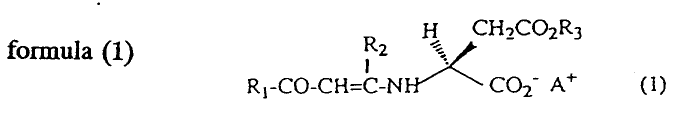
(Wherein R₁ is a alkyl or alkoxy group with 1 to 4 carbon atoms or an aromatic group with 6 to 10 carbon atoms; R₂ is a alkyl group with 1 to 4 carbon atoms or a aromatic group with 6 to 10 carbon atoms; R₃ is a alkyl group with 1 to 4 carbon atoms or a benzyl group; A is a protonated tertiary amine or dicyclohexylamine or an alkaline metal). 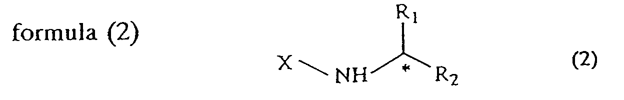
(Wherein R₁ is an alkyl group with 1 to 6 carbon atoms or an alkoxymethyl group with 2 to 7 carbon atoms; R₂ is a benzyl group, a cyclohexyl group, a cyclohexylmethyl group, a phenyl group, or a phenyl group with a substituent in its 2-,3-or 4-position selected among the group consisting of F, Cl, Br, I, a hydroxyl group, a alkoxy group with 1 to 6 carbon atoms, a cyano group, a nitro group, a acetyl group, a amino group, an acetyl amino group, or a phenyl group with a methylenedioxy group, a trimethylene group, a tetramethylene group in its 2,3- or 3,4- position, or a 2-,3- or 4-pyridyl group, a 2- or 3-furyl group, or a 2- or 3-thienyl group; the molecular structure containing C* is (S) or (RS) when R₁ is a alkyl group; (R), (S) or (RS) when R₁ is a alkoxymethyl group. X is a D-α-amino acid residue or a DL-α amino acid residue selected among the group consisting of D-norleucine, D-leucine, D-isoleucine, D-alloisoleucine, D-threonine, D-O-methylthreonine, D-allothreonine, D-O-methylallothreonine, D-or DL-furylglycine, or cyclic or non-cyclic α,α-dialkyl amino acid residue with 3 to 6 carbon atoms when R₁ is a alkyl group and R₂ is a benzyl group, a cyclohexyl group, a cyclohexylmethyl group, or phenyl group; X is a D-α-amino acid residue or a DL-α-amino acid residue selected among the group comprising D-alanine, D-α-aminobutyric-acid, D-norvaline, D-valine, D-norleucine, D-leucine, D-isoleucine, D-alloisoleucine, D-t-leucine, D-serine, D-O-methylserine, D-threonine, D-O-methylthreonine, D-allothreonine, D-O-methylallothreonine, D-phenylglycine, D-or DL-furylglycine, or a cyclic or non-cyclic α,α-dialkylamino acid residue with 3 to 6 carbon atoms when R₁ is an alkyl group and R₂ is a phenyl group with a substituent in its 2-, 3- or 4-position selected among the group consisting of F, Cl, Br, I, a hydroxyl group, an alkoxy group with 1 to 6 carbon atoms, a cyano group, a nitro group, an acetyl group, an amino group, an acetylamino group, or a phenyl group with a methylenedioxy group, a trimethylene group or a tetramethylene group in its 2, 3- or 3,4-position, or 2-, 3- or 4-pyridyl group, a 2- or 3- furyl group, or a 2- or 3-thienyl group when R₁ is an alkoxymethyl group, L-Asp and X are α-bonded).

### DETAILED DESCRIPTION OF THE INVENTION

The aspartic acid derivative of formula (1) is produced by mixing L-aspartic acid β-alkyl ester with "A" base and β-diketones or acetoacetic acid esters, as amino protecting groups in an appropriate solvent. The appropriate solvent may be water, alcohols such as methanol and ethanol, ethers such as tetrahydrofuran and 1,4- dioxane, nitriles such as acetonitrile, dimethylformamide, dimethylsulfoxide and other mixed solvents.

L-aspartic acid β-alkylester can be easily obtained by treating L-aspartic acid with thionyl chloride in corresponding alkyl alcohol (H. Schwarz et.al., J. Am. Chem. Soc., 79, 5697 (1957)) or in alkyl alcohol containing hydrogen halogenide (D.W. Coleman et. al., J. Chem. Soc., 1951, 2294) etc.

A represents tertiary amines such as triethylamine, tributylamine, trioctyl amine and N-methylmorpholine, secondary amines such as dicyclohexylamine with a bulky substituent and weak nucleophilicity, and inorganic bases consisting of alkaline metals such as sodium hydroxide, potassium hydroxide, and sodium carbonate. Tertiary amines such as tributylamine and trioctylamine are especially recommended because of their slurry properties in the next process step.

An amino-protecting group includes acetoacetic acid esters such as methyl acetoacetate and ethyl acetoacetate, and β-diketones such as acetylacetone and benzoylacetone.

The aspartic acid derivative of formula (1) produced by mixing said three substances coexists with water which will be produced in reaction. A protic solvent such as water and alcohol interferes with the reaction, so it needs to be removed in an appropriate manner before the reaction with alkyl chloroformate begins in the next process step. By distilling off water and alcohol, the reaction solution can be concentrated. When the substances solidify in the reaction solution, the solid can be separated from the liquid by filtering or other methods.

To produce mixed anhydride from the compound of formula (1) and alkyl chloroformate, the compound of formula (1) may be dissolved or suspended in an aprotic solvent, then alkyl chloroformate can be added to obtain a mixed anhydride.

The aprotic solvent includes aromatic hydrocarbons such as benzene and toluene, acetic acid esters such as ethyl acetate and butyl acetate, ketones such as acetone and methylethylketone, hydrocarbon halides such as dichloromethane and chloroform, and ethers such as tetrahydrofuran and dioxane. Generally, aromatic hydrocarbons and acetic acid esters are ideal in terms of liquid properties of the reaction solution and of the next deprotecting process.

It is desirable for the reaction to take place at a low temperature to suppress side reactions. The temperatures that bring the best yields are ≦5°C, preferably ≦0°C and ideally ≦-10°C.

It is economical that the mixing molar ratio of the compound of formula (1) and alkyl chloroformate is 1 ( equimolar ), however, the molar ratio range of 0.6 to 1.5 is acceptable.

The alkyl chloroformate includes methyl chloroformate, ethyl chloroformate, isobutyl chloroformate, and benzyl chloroformate.

By mixing the reaction solution containing the resulting mixed anhydride with amino acid derivative, the protected α-L-aspartyldipeptide-amide derivative of formula (5) is obtained easily. 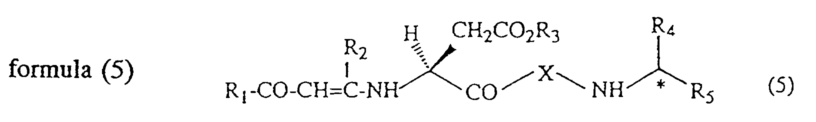
(Wherein R₁ is an alkyl or alkoxy group with 1 to 4 carbon atoms or aromatic group with 6 to 10 carbon atoms; R₂ is an alkyl group with 1 to 4 carbon atoms or aromatic group with 6 to 10 carbon atoms; R₃ is an alkyl group with 1 to 4 carbon atoms or benzel group; R₄ is an alkyl group with 1 to 6 carbon atoms or alkoxymethyl group with 2 to 7 carbon atoms; R₅ is a benzyl, cyclohexyl, cyclohexylmethyl and phenyl groups; or a phenyl group with a substituent in its 2-, 3- or 4-position selected among the group comprising F, Cl, Br, I, a hydroxy group, an alkoxy group with 1 to 6 carbon atoms, a cyano group, a nitro group, an acetyl group, an amino group and acetylamino group; or a phenyl group substituted with a methylenedioxy group, trimethylene group or tetramethylene group in its 2,3- or 3,4 position, or 2-, 3- or 4-pyridyl, 2- or 3-furyl, or 2- or 3-thienyl group. The configuration of C* is (S) or (RS) when R₄ is an alkyl group; (R), (S) or (RS) when R₄ is an alkoxymethyl group.
X is a D-α-amino acid residue or a DL-α amino acid residue selected among the group comprising D-norleucine, D-leucine, D-isoleucine, D-alloisoleucine, D-threonine, D-O-methylthreonine, D-allothreonine, D-O-methylallothreonine, D-or DL-furylglycine, or cyclic or non-cyclic α,α-dialkyl amino acid residue with 3 to 6 carbon atoms when R₄ is an alkyl group and R₅ is a benzyl group, a cyclohexyl group, a cyclohexylmethyl group, or a phenyl group; X is a D-α-amino acid residue or or a DL-α-amino acid residue selected among the group comprising of D-alanine, D-α-aminobutyric acid, D-norvaline, D-valine, D-norleucine, D-leucine, D-isoleucine, D-alloisoleucine, D-t-leucine, D-serine, D-O-methylserine, D-threonine, D-O-methylthreonine, D-allothreonine, D-O-methylallothreonine, D-phenylglycine, D-or DL-furylglycine, or a cyclic or non-cyclic α,α-dialkylamino acid residue with 3 to 6 carbon atoms when R₄ is an alkyl group and R₅ is a phenyl group with a substituent in its 2-, 3- or 4-position selected among the group comprising F, Cl, Br, I, hydroxyl, alkoxy groups with 1 to 6 carbon atoms, cyano, nitro, acetyl, amino, acetylamino groups, or a phenyl group with a methylenedioxy group, trimethylene group or tetramethylene group in its 2,3- or 3,4- position, or 2-, 3- or 4-pyridyl group, a 2- or 3- furyl group, or a 2- or 3-thienyl group, and when R₄ is an alkoxymethyl group, L-Asp and X are α-bonded.)

The amino acid amide derivative in this invention can be obtained by removing the protective group from N-benzyloxycarbonyl or N-tert-butoxycarbonyl amino acid derivatives, as disclosed in US Patent 5286509; it can also be obtained by using the organic layer obtained initially by reacting with alkylchloroformate mixed anhydride of amino acid derivatives of formula (3), condensing the product with amine derivatives of formula (4), then treating it in acidic water to obtain an aqueous solution containing amino acid amide derivatives for extraction with organic solvent under alkaline conditions. 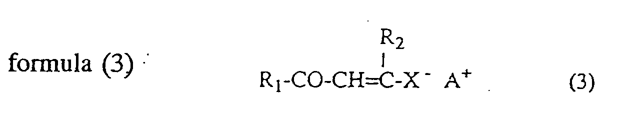
(Wherein R₁ is a alkyl or alkoxy group with 1 to 4 carbon atoms or an aromatic group with 6 to 10 carbon atoms; R₂ is an alkyl group with 1 to 4 carbon atoms or a aromatic group with 6 to 10 carbon atoms; X is a D-α-amino acid residue or a DL-α-amino acid residue selected among the group comprising D-alanine, D-α-amino butyric acid, D-norvaline, D-valine, D-norleucine, D-leucine, D-isoleucine, D-alloisoleucine, D-t-leucine, D-serine, D-O-methylserine, D-threonine, D-O-methylthreonine, D-allothreonine, D-O-methylallothreonine or D-phenylglycine, D- or DL-furylglycine, or a cyclic or non-cyclic α,α-dialkyl amino acid residue with 3 to 6 carbon atoms;
A represents protonated tertiary amine, dicyclohexylamine, or an alkaline metal.) 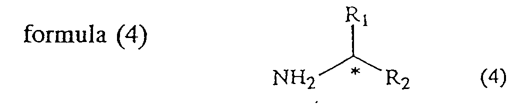
(Wherein R₁ is an alkyl group with 1 to 6 carbon atoms or an alkoxymethyl group with 2 to 7 carbon atoms; R₂ is a benzyl group, a cyclohexyl group, a cyclohexylmethyl group, a phenyl group, or a phenyl group with a substituent in its 2-, 3- or 4-position selected from F, Cl, Br, I, a hydroxyl group, an alkoxy group with 1 to 6 carbon atoms, a cyano group, a nitro group, an acetyl group, a amino group, an acetylamino group, or a phenyl group with a methylenedioxy group, a trimethylene group, a tetramethylene group in its 2, 3- or 3,4- position, or a 2-, 3- or 4-pyridyl group, a 2- or 3- furyl group, or a 2- or 3-thienyl group; the configuration of C* is (S) or (RS) when R₁ is an alkyl group; (R) , (S) or (RS) when R₁ is an alkoxymethyl group.

In this process, conditions for mixed anhydride reaction and condensation reactions remain the same as described previously: the same sub-materials such as alkyl chloroformate ad tertiaryamine, and the temperature are used both for condensing amine derivatives of formulas (4) with amino acid derivatives and for resulting condensed compound with aspartic acid component. This means that the method is very advantageous industrially due to the common use of identical storage tanks and reaction vessels.

In the resulting mixture, protected α-L-aspartyl-depeptide amide derivatives exist. The procedure to obtain the target α-L-aspartyldipeptide amide derivative by removing the protecting group is as follows: add mineral acid solution such as diluted hydrochloric acid to the reaction mixture to subject it to acidic conditions at room temperature from 30 minutes to several hours. In this way, the amino protecting group can be readily cleaved, resulting in α-L-aspartyldipeptide amide β-alkylester of formula (6). When a solvent in which the compound of formula (5) is poorly soluble is employed, this substance may be present as a crystal or in the form of a solid. In such a case, a solid/liquid separation procedure such as filtration is conducted to obtain the solid to which then dilute hydrochloric acid is added to yield the substance correponding to formula (6). 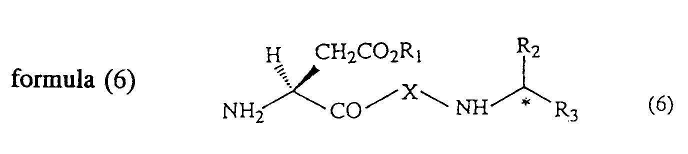
(Wherein R₁ is a alkyl group with 1 to 4 carbon atoms or an benzyl group; R₂ is an alkyl group with 1 to 6 carbon atoms, or alkoxymethyl group with 2 to 7 carbon atoms, R₃ is a benzyl, cyclohexylmethyl, phenyl group, or a phenyl group with a substituent in its 2-, 3- or 4-position selected from F, Cl, Br, I, a hydroxyl group, an alkoxy group with 1 to 6 carbon atoms, cyano, nitro, acetyl, amino, acetyl amino groups, or a phenyl group with a methylenedioxy, trimethylene, or tetramethylene group in its 2, 3- or 3, 4- position, or a 2-, 3- or 4-pyridyl, 2- or 3- furyl group, or 2- or 3-thienyl group; the configuration of C* is (S) or (RS) when R₂ is an alkyl group; (R), (S) or (RS) when R₂ is an alkoxymethyl group;
X is a D-α-amino acid residue or a DL-α amino acid residue selected among the group comprising D-norleucine, D-leucine, D-isoleucine, D-alloisoleucine, D-threonine, D-O-methylthreonine, D-allothreonine, D-O-methylallothreonine, D-or DL-furylglycine, or cyclic or non-cyclic α,α-dialkyl amino acid residue with 3 to 6 carbon atoms when R₂ is an alkyl group and R₃ is a benzyl, cyclohexyl, a cyclohexylmethyl, or phenyl group;
a D-α-amino acid residue or a DL-α-amino acid residue selected among the group comprising D-alanine, D-α-aminobutyric acid, D-norvaline, D-valine, D-norleucine, D-leucine, D-isoleucine, D-alloisoleucine, D-t-leucine, D-serine, D-O-methylserine, D-threonine, D-O-methylthreonine, D-allothreonine, D-O-methylallothreonine, D-phenylglycine, D-or DL-furylglycine, or a cyclic or non-cyclic α,α-dialkylamino acid residue with 3 to 6 carbon atoms when R₂ is a alkyl group, and R₃ is a phenyl group with a substituent in its 2-, 3- or 4-position selected among the group comprising F, Cl, Br, I, a hydroxyl group, an alkoxy group with 1 to 6 carbon atoms, a cyano group, a nitro group, an acetyl group, an amino group, an acetylamino group, or a phenyl group with a methylenedioxy group, a trimethylene group or a tetramethylene group in its 2, 3- or 3, 4- position, or 2-, 3- or 4-pyridyl group, a 2- or 3- furyl group, or a 2- or 3-thienyl group, and, when R₂ is an alkoxymethy group, L-Asp and X are α-bonded.)

The substance of formula (6) is hydrolyzed under acidic conditions to convert the β-ester in aspartic acid into carboxylic acid and produce the objective α-L-aspartyldipeptide amide derivatives. This reaction requires more severe process conditions than simply removing the amino protecting group. The required acid concentration is from 1N to 6N hydrochloric acid and the temperature is from room temperature to 80°C. The process time is between 30 minutes and 24 hours to hydrolyze esters without cleaving the amide bond of the target substance. For example, the higher acid concentration can be combined with a relatively low temperature and shorter time.

When benzyl ester is in the β-position, the ester group can be removed by catalytic reduction. Remove the amino protecting group from the acid solution, then add a catalyst such as Pd-Carbon to achieve the conversion into carboxylic acid while stirring under hydrogen atmosphere. Then, after removal of the catalyst by filtration, the mixture is neutralized to obtain the target α-L-aspartyldipeptide amide derivatives.

Removal of the amino protecting group and hydrolysis of the esters can take place at the same time. It is preferable that the compounds of formula (5) are separated from the reaction solution by concentrating or filtering when using solvents such as ethyl acetate which can easily be hydrolyzed. The resulting compound of formula (5) can be exposed to the ester hydrolysis conditions to remove the amino protecting group at the same time. When using unhydrolyzable solvents such as toluene, the condensation reaction solution can be exposed to hydrolytic conditions.

The resulting acidic solution containing α-L-aspartyldipeptide amide derivatives can be neutralized with bases such as sodium hydroxide and then crystallization can be controlled by concentration or cooling to obtain the objective α-L-aspartyldipeptide amide derivatives in crystal form.

### EXAMPLES

The following examples will describe the invention in detail. The HPLC conditions are as follows. Column: Inertsil ODS-2, 6Φ × 150mm; eluent: 0.1M KH₂ PO₄ (pH3.0) / MeCN = 80/20 (V/V); flow rate: 1ml/min; temperature: room temperature; detection: UV (210nm).

### Example 1

1.27g (6.90 mmol) of L-aspartic acid β-methyl ester hydrochloride, 0.80g (6.9mmol) of methyl acetoacetate, 4.88g (13.8mmol) of trioctyl amine were added to 20 ml of methanol (Solution I); Solution I was refluxed for three hours, then concentrated under reduced pressure. 27ml of ethyl acetate was added to the residue (syrup) and was cooled to below - 10°C while stirring, and 10ml of ethyl acetate solution containing 0.66ml (6.9mmol) of ethyl chloroformate was added while maintaining the temperature of the reaction solution at -11°C to -12°C. 20 minutes later, 15ml of ethyl acetate solution containing 1.37g (5.85mmol) of D-valine-N-(S)-α-ethylbenzylamide was added to the reaction solution while maintaining the temperature at -13°C to -17°C. The reaction mixture was warmed to room temperature ad 20ml of 1N-HCl was added and stirred for one hour at the room temperature. After the solution was separated into layers, the ethyl acetate layer was extracted twice with diluted hydrochloric acid. The aqueous layer was collected, HPLC analyzed, and measured 1.81g (4.97mmol) of α-L-aspartyl-D-valine-N-(S)-α-ethylbenzylamide -methyl ester. The yield was 85.0%.

The aqueous layer was concentrated, 12ml of 2N-HCl was added to the residue and kept at 60°C for five hours. The hydrolyzed solution was HPLC analyzed and measured 1.55g (4.45 mmol) of α-L-aspartyl-D-valine-N-(S)-α-ethylbenzylamide. The yield was 89.4%.

The hydrolyzed solution pH was adjusted to pH 6 with 25% NaOH, and the resulting slurry was stirred at 5°C overnight and filtered at reduced pressure. The resulting α-L-aspartyl-D-valine-N-(S)-α-ethylbenzylamide crystals were rinsed with a small amount of water and dried at reduced pressure. The gained amount was 1.42g. The crystallization yield was 91.4%.

### Example 2

5.85g (50.0mmol) of D-valine, 5.81g (50.0mmol) of methyl acetoacetate and 9.05g (50.0mmol) of dicyclohexylamine were added to 50ml of methanol (solution II). Solution II was heated to reflux for three hours and concentrated at reduced pressure. 165ml of ethyl acetate and 0.48g (4.7mmol) of N-methylmorphorine were added to the residue and stirred while cooling, and 60ml of ethyl acetate solution containing 6.18ml (4.72 mmol) of isobutyl chloroformate was added while maintaining temperature at - 17°C to -20°C. 20 minutes later, 100ml of ethyl acetate solution containing 5.42g (40.1mmol) of (S)--ethylbenzylamine was also added to the mixture while maintaining the temperature as it is. The mixture was warmed to the room temperature and then filtered to remove precipitated dicyclohexylamine hydrochloride crystals ad 100ml of 1N HCl was added to the filtrate. The mixture was stirred at the room temperature for ninety minutes and was separated into layers. The aqueous layer was adjusted to pH11 with 1N-NaOH and extracted with approximately 200ml of ethyl acetate. The resulting 226ml of ethyl acetate layer contained 6.63g (28.3mmol) of D-valine-N-(S)-α-ethylbenzylamide. The yield was 70.5%.

3.06g (16.7mmol) of L-aspartic acid -methyl ester hydrochloride, 2.25g (19.4mmol) of methyl acetoacetate and 6.51g (35.9mmol) of dichlorohexylamine were added to 30ml of methanol, then refluxed while heating for three hours; the reaction mixture was concentrated at reduced pressure.

Ethyl acetate was added to the residue and the filtered to remove precipitated dicyclohexylamine hydrochloride. The filtrate was concentrated to obtain 8.38g of syrup-like residue. 50ml of ethyl acetate and 0.85g (8.4mmol) of N-methylmorpholine were added to the residue, and the resulting mixture was cooled as described above, ad ethyl acetate solution containing 2.17ml (16.7mmol) of isobutyl chloroformate was added. 20 minutes later, about 35ml of the concentrate made from 120ml of the ethyl acetate layer (containing 15mmol of D-valine-N-(S)-α-ethylbenzylamide) was added.
The temperature was raised to the room temperature ad filtered at reduced pressure to obtain deposited crystals. The crystals were hydrolyzed with 100 ml of 0.5N-HCl at the room temperature for two hours and then filtered to remove precipitated dicyclohexylamine hydrochloride and the filtrate was HPLC analyzed and measured 3.60g (9.91mmol, yield: 66.0%) of α-L-aspartyl-D-valine-N-(S)-α-ethylbenzylamide β-methyl ester and 0.116g (0.332mmol, yield: 2.2%) of α-L-aspartyl-D-valine-N-(S)-α-ethylbenzylamide. The aqueous layer, obtained by mixing the mother liquor with diluted hydrochloric acid ad separating into layers, was HPLC analyzed and measured 0.545g (1.50mmol, yield: 10.0%) of α-L-aspartyl-D-valine-N-(S)-α-ethylbenzylamide methyl ester and 26mg (0.073mmol; yield: 0.5%) of α-L-aspartyl-D-valine-N-(S)-α-ethylbenzylamide. The total condensation yield was 78.7%. The above two hydrolyzed solutions were put together and concentrated, and 200ml of 2N-HCl were added to the residue and kept at 60°C for four hours, and then 3.58g (10.26mmol, yield: 86.9%) of α-L-aspartyl-D-valine-N-(S)-α-ethylbenzyl amide was measured.

### Example 3

1.84g (10.0mmol) of L-aspartic acid β-methyl ester hydrochloride, 1.00g (10.0mmol) of acetylaceton and 3.63g (20.0mmol of dicyclohexylamine were added to 40 ml of methanol (Solution III); Solution III was refluxed while heating for three hours and concentrated at reduced pressure. 50ml of ethyl acetate was added to the resulting syrup-like residue and was cooled to < -10°C and stirred; 11ml of ethyl acetate solution containing 0.96ml (10mmol) of ethyl chloroformate was added while maintaining the reaction solution temperature at -11°C to -12°C. 20 minutes later, 45ml of ethyl acetate solution containing 1.91g (9.27mmol) of D-α-aminobutyric acid-N-(S)-α-methyl benzyl amide was added while maintaining the reaction solution temperature at -13°C to -17°C. The solution was warmed to the room temperature and them filtered to remove dicyclohexylamine hydrochloride and the filtrate was concentrated; acetonitrile and 40ml of 2N-HCl were added to the residue, and then heated at 60°C for four hours. The resulting solution was HPLC analyzed and measured 1.79g (5.58mmol) of α-L-aspartyl-D-α-aminobutyric acid-N-(S)-α-methylbenzylamide. The yield was 60.6%.

### Example 4

1.02g (5.00mmol) of L-aspartic acid β-benzyl ester, 0.63ml (5.0mmol) of ethyl acetoacetate and 0.909g (5.00mmol) of dicyclohexylamine were added to 30ml of methanol (Solution IV); Solution IV was stirred at the room temperature overnight and concentrated at reduced pressure. 22ml of ethyl acetate and 0.3 ml of triethylamine were added to the residue, ad the mixture was stirred while cooling below - 10°C; 7ml of ethyl acetate solution containing 0.47ml (4.94mmol) of ethyl chloroformate was added while maintaining the reaction solution temperature at -20±5°C. 30 minutes later, approximately 6ml of the concentrate made of 34ml of the ethyl acetate layer of Example 2 containing 4.22mmol of (D-valine-N-(S)-α-ethylbenzyl amide) was added to the reaction solution while maintaining the temperature at - 20±3°C. The mixture was warmed to room temperature and then filtered to remove dicyclohexylamine hydrochloride and 11ml of 1NHCl was added to the filtrate, and the mixture was stirred at room temperature for one hour and concentrated. 40 ml of acetic acid was added to the syrup-like residue, and then 0.36g of 10% PdC was added, and the mixture was stirred for three days under hydrogen atomosphere for catalytic reduction. The mother liquor, after catalyst removal, was HPLC analyzed and measured 0.592g (1.70mmol) of α-L-aspartyl-D-valine-N-(S)- α-ethylbenzyl amide. The yield was 40.2%.

### Example 5

1.52g (13.0mmol) of D-valine, 1.51g (13.0mmol) of methyl acetoacetate, 2.36g (13.0mmol) of dicyclohexylamine were added to 13ml of methanol; the solution was refluxed while heating for three hours and concentrated at reduced pressure. 45ml of ethyl acetate and 0.13g (1.3mmol) of N-methylmorpholine were added to the residue ad stirred and cooled (Solution V);15ml of ethyl acetate solution containing 1.71ml (13.0mmol) of isobutyl chloroformate was added to Solution V while maintaining the temperature at - 15°C to - 17°C. 20 minutes later, 25 ml of ethyl acetate solution containing 1.59g (10.5mmol) of (R)-α-methoxymethylbenzylamine was added while maintaining the same temperature. The solution was warmed to room temperature, and then, filtered to remove precipitated dicyclohexylamine hydrochloride. 25ml of 1N HCl was added to the filtrate and the solution was then stirred for ninety minutes at room temperature to separate into layers. The resulting aqueous layer was adjusted to pH11 with 1N NaOH and the mixture was extracted with approximately 50ml of ethyl acetate. The resulting ethyl acetate layer was dried with anhydrous magnesium sulfate; the mixture was filtered and the filtrate was concentrated at reduced pressure, and then it was dried to obtain 2.16g (8.63mmol) of D-valine-N-(R)-α-methoxymethylbenzylamide in form of clear light yellow syrup. The yield was 82.2%.

1.84g (10.0mmol) of L-aspartic acid β-methyl ester hydrochloride, 1.16g (10.0mmol) of methyl acetoacetate and 3.63g (20.0mmol) of dicyclohexylamine were added to 18ml of methanol; then the mixture was refluxed while heating for three hours and concentrated at reduced pressure. Ethyl acetate was added to the residue and then filtered to remove precipitated dicyclohexylamine hydrochloride. The filtrate was concentrated to obtain 4.74g of syrup-like residue. 30ml of ethyl acetate and 0.51g (5.0mmol) of N-methylmorpholine were added to the residue and was cooled as described above, ad ethyl acetate solution containing 1.31ml (10.0mmol) of isobutyl chloroformate was added. 20 minutes later, the solution made by dissolving 2.13g (8.52mmol) of the D-valine-N-(R)-α-methoxymethylbenzylamide into 20ml of ethyl acetate was added to the mixture. The solution was warmed to room temperature and was concentrated at reduced pressure to distill ethyl acetate off. Acetonitrile and 200ml of 2N HCl were added to the residue ad heated at 60°C for twenty hours. The solution was HPLC analyzed and measured 2.49g (6.81mmol) of α-L-aspartyl-D-valine-N-(R)-α methoxymethylbenzyl amide. The yield was 79.9%.

The hydrolized solution was adjusted to pH6 with 25% NaOH and was concentrated at reduced pressure; the resulting slurry was cooled and filtered at reduced pressure. The resulting α-L-aspartyl-D-valine-N-(R)-α methoxymethylbenzylamide crystals were rinsed with a small amount of water, ethyl acetate and chloroform and was dried under reduced pressure. The gained amount was 2.00g. The crystalization yield was 80.3%.

### Example 6

1.47g (8.0mmol) of L-aspartic acid -methyl ester hydrochloride, 0.93g (8.0mmol) of methyl acetoacetate and 5.66g (16.0mmol) of trioctyl amine were added to 25ml of methanol (Solution VI); Solution VI was refluxed while heating for three hours and was concentrated at reduced pressure. 30ml of ethyl acetate was added to the resulting syrup-like residue and was stirred while cooling to <-10°C; 15ml of ethyl acetate solution containing 0.77ml (8.0mmol) of ethyl chloroformate was added to the mixture while maintaining the temperature at -11°C to -12°C. 20 minutes later, 20ml of ethyl acetate solution containing 1.79g (6.78mmol) of D-valine-N-(R)-α-ethoxymethylbenzylamide was added while maintaining the temperature at -13°C to - 17°C. The solution was warmed to room temperature and 25ml of 1N HCl was added to the reaction solution and the solution was stirred at room temperature for one hour.
After layers were separated, the ethyl acetate layer was extracted twice with diluted hydrochloric acid solution. The aqueous layer was concentrated and 15ml of 2N HCl was added to the residue, and the mixture was heated at 60C for eight hours. The hydrolyzed solution was HPLC analyzed and measured 2.25g (5.94mmol) of α-L-aspartyl-D-valine-N-(R)-α-ethoxymethylbenzylamide. The yield was 87.6%.

The hydrolyzed solution was adjusted to pH6 with 25% NaOH and the resulting slurry was stirred at 5C overnight, and then filtered at reduced pressure. The resulting α-L-aspartyl-D-valine-N-(R)-α-ethoxymethylbenzylamide crystals were rinsed with a small amount of water and dried under reduced pressure. The gained amount was 2.01g. The cryslallization yield was 89.4%.

### Example 7

1.84g (10.0mmol) of L-aspartic acid β-methyl ester hydrochloride, 1.00g (10.0mmol) of acetyl acetone and 3.63g (20.0mmol) of dicyclohexylamine were added to 40ml of methanol (Solution VII); Solution VII was refluxed while heating for three hours and concentrated at reduced pressure. 50ml of ethyl acetate was added to the resulting syrup-like residue and stirred while cooling to <-10°C; 15ml of ethyl acetate solution containing 0.96ml (10.0mmol) of ethyl chloroformate was added while maintaining the temperature at -11°C to -12°C. 20 minutes later, 40ml of ethyl acetate solution containing 2.15g (9.11mmol) of D-α-aminobutyric acid-N-(R)-α-methoxymethylbenzyl amide was added to the reaction solution while maintaining the temperature at -13°C to - 17°C. The solution temperature was raised to the room temperature and the mixture was filtered to remove dicyclohexylamine hydrochloride. The filtrate was concentrated; acetonitrile and 40 ml of 2N HCl were added to the residue ad the mixture was heated at 60C for five hours. The solution was HPLC analyzed and measured 2.16g (6.16mmol) of α-L-aspartyl-D-α-aminobutyric acid-N-(R)-α-methoxymethylbenzylamide. The yield was 67.6%.

### Example 8

2.04g (10.0mmol) of L-aspartic acid β-benzylester, 1.26ml (10.0mmol) of ethyl acetoacetate and 1.82g (10.0mmol) of dicyclohexylamine were added to 50ml of methanol (Solution VIII); Solution VIII was stirred at room temperature overnight and concentrated at reduced pressure. 50ml of ethyl acetate and 0.6ml of triethylamine were added to the resulting residue, and the solution was stirred while cooling to <-10°C; 15ml of ethyl acetate solution containing 0.95ml (10.0mmol) of ethyl chloroformate was added while maintaining the temperature at -13°C to -17°C. 30 minutes later, 15ml of ethyl acetate solution containing 2.38g (9.51mmol) of D-valine-N-(R)-α-methoxymethylbenzylamide was added to the solution while maintaining the temperature at -13°C to -17°C. The solution temperature was
raised to room temperature and the mixture was filtered. 25ml of 1N HCl was added to the filtrate, and the mixture was stirred at the room temperature for one hour and concentrated. 80ml of acetate was added to the syrup-like residue, and 0.75g of 10% Pd-C was added, and the mixture was stirred under hydrogen atomosphere for three days for catalytic reduction. The mother liquor, after catalyst removal by filtering, was HPLC analyzed and measured 1.83g (5.01mmol) of α-L-aspartyl-D-valine-N-(R)-α-methoxylbenzylamide. The yield was 52.7%.

### Example 9

2.00g (19.4mmol) of D-α-aminobutyric acid, 2.25g (19.4mmol) of methyl acetoacetate, and 3.52g (19.4mmol) of dicyclohexylamine were added to 17ml of methanol and was refluxed while heating for three hours and the mixture was concentrated at reduced pressure. 60ml of ethyl acetate and 0.20g (1.9mmol) of N-methylmorpholine were added, the mixture was stirred while cooling (Solution IX); 20ml of ethyl acetate solution containing 2.55ml (19.4mmol) of isobutyl chloroformate was added to Solution IX while maintaining the temperature at -15°C to -17°C. 20 minutes later, 30ml of ethyl acetate solution containing 2.37g (15.7mmol) of (S)-α-ethyl-p-hydroxybenzylamine was added at the same temperature. The solution was warmed to the room temperature and filtered. 30ml of 1N HCl was added to the filtrate at the room temperature and stirred for ninety minutes, and then separated into layers. The resulting aqueous layer was adjusted to pH11 with 1N NaOH, and then was extracted with approximately 70 ml of ethyl acetate.
The resulting ethyl acetate layer was dried over anhydrous magnasium sulfate, which was filtered later; the filtrate was concentrated at reduced pressure and dried. 2.72g (11.5mnol) of D-α-aminobutyric acid-N-(S)-α-ethyl-p-hydroxy benzylamide was measured as clear light yellow syrup. The yield was 73.2%.

2.74g (14.9 mmol) of L-aspartic acid β-methyl ester hydrochloride, 1.73g (14.9 mmol) of methyl acetoacetate and 5.41g (29.8 mmol) of dicyclohexylamine were added to 25ml of methanol; the mixture was refluxed while heating for three hours, and then concentrated at reduced pressure. Ethyl acetate was added to the resulting residue and the mixture was filtered. The filtrate was concentrated ,and then the syrup-like residue was obtained. 45ml of ethyl acetate and 0.75g (7.5 mmol) of N-methylmorpholine were added and cooled as described above, and ethyl acetate solution containing 1.96ml (14.9 mmol) of isobutyl chloroformate was added. 20 minute later, the solution made by dissolving 2.67g (11.3 mmol) of D-α-aminobutyric acid-N-(S)-α-ethyl-p-hydroxyhenzylamide into 30ml of ethyl acetate was added to the mixture. The solution was warmed to
room temperature and ethyl acetate was distilled off by concentrating the solution at reduced pressure.
Acetonitrile and 200ml of 2N HCl were added to the residue and heated at 60C for ten hours. The solution was HPLC analyzed and measured 2.94g (8.37 mmol) of α-L-aspartyl-D-α-aminobutyric acid-N-(S)-α-ethyl-p-hydroxybenzylamide. The yield was 74.1%.
The hydrolyzed solution was adjusted to pH6 with 25% NaOH and concentrated under reduced pressure; the resulting slurry was ice cooled and filtered at reduced pressure. The resulting α-L-aspartyl-D-α-amino butyric acid-N-(S)-α-ethyl-p-hydroxybenzylamide crystals were rinsed with a small amount of water, ethyl acetate, and chloroform, and then dried under reduced pressure. The gained amount was 2.43g. The crystallization yield was 82.7%.

### Example 10

1.47g (8.00 mmol) of L-aspartic acid β-methyl ester hydrochloride, 0.93g (8.0 mmol) of methyl acetoacetate ad 5.66g (16.0 mmol) of trioctylamine were added to 25ml of methanol, and refluxed while heating for three hours (Solution X); Solution X was concentrated at reduced pressure. 30ml of ethyl acetate was added to the resulting syrup-like residue and stirred while cooling to ≦ -10°C. 15ml of ethyl acetate solution containing 0.77ml (8.0 mmol) of ethyl chloroformate was added to the reaction mixture while maintaining the temperature at -11°C to -12°C. 20 minutes later, 20ml of ethyl aceate solution containing D-valine-N-(S)-α-ethyl-p-hydroxybenzylamide was added while maintaining the temperature at -13°C to -17°C. The solution was warmed to the room temperature, and 25ml of 1N HCl was added to the solution and stirred at the room temperature for one hour. After the solution was separated into layers, the ethyl acetate layer was extracted twice with diluted hydrochloric acid. The aqueous layer was concentrated ad 15ml of 2N HCl was added to the residue, and heated at 60°C for five hours. The hydrolyzed solution was HPLC anyalyzed and measured 1.70g (4.66 mmol) of α-L-aspartyl-D-valine-N-(S)-α-ethyl-p-hydroxybenzylamide. The yield was 69.9%.

The hydrolyzed solution was adjusted to pH6 with 25% NaOH; the resulting slurry was stirred at 5°C overnight ad filtered under reduced pressure. The resulting α-L-aspartyl-D-valine-N-(S)-α-ethyl-p-hydroxyherzylamide crystals were rinsed with a small amount of water and dried under reduced pressure. The gained amount was 1.51g. The crystallization yield was 88.8%.

### Example 11

2.00g (17.1mmol) of D-valine, 1.98g (17.1 mmol) of methyl acetoacetate, 3.10g (17.1 mmol) of dicyclohexylamine were added to 17ml of methanol and refluxed while heating for three hours (Solution XI); Solution XI was concentrated at reduced pressure. 60ml of ethyl acetate, 0.17g (1.7 mmol) of N-methylmorpholine were added to the resulting residue and stirred while cooling; 20ml of ethyl acetate solution containing 2.24ml (17.1 mmol) of isobutyl chloroformate was added to the mixture while maintaining the temperature at -15C to -17C. 20 minutes later, 30ml of ethyl acetate solution containing 2.34g (13.8 mmol) of (S)-α-ethyl-p-chlorobenzylamine was added to the mixture. The solution was warmed to the room temperature and filtered. 30ml of 1N HCl was added to the filtrate and the solution was stirred at the room temperature for 90 minutes and then separated into layers. The resulting aqueous layer was adjusted to pH11 with 1N NaOH ad extracted with approximately 60ml of ethyl acetate. The resulting ethyl acetate layer was dried over anhydrous magnesium sulfate and filtered to remove magnesium sulfate; the filtrate was concentrated at reduced temperature and dried, and then 3.14g (11.7 mmol) of D-valine-N-(S)-α-ethyl-p-chlorobenzylamide of a clear light yellow syrup-like was measured. The yield was 84.8%.

2.41g (13.1 mmol) of L-asparitic acid β-methyl ester hydrochloride, 1.52g (13.1 mmol) of methyl acetoacetate, and 4.75g (26.2 mmol) of dicyclohexylamine were added to 25ml of methanol, refluxed while heating for three hours, and then concentrated at reduced pressure. Ethyl acetate was added to the resulting residue and then the mixture was filtered. The filtrate was concentrated, and then a syrup-like residue was obtained. 40ml of ethyl acetate and 0.66g (6.6mmol) of N-methylmorpholine were added to the residue and cooled as described above, then 1.72ml (13.1mmol) of isobutyl chloroformate was added. 20 minutes later, the solution made by dissolving 3.01g (11.2mmol) of D-valine-N-(S)-α-ethyl-p-chlorobenzylamide was into 25ml of ethyl acetate, was added to the mixture. The resulting solution was warmed to the room temperature and ethyl acetate was distilled off by concentrating the reaction solution under reduced pressrue. Acetonitrile and 200ml of 2N HCl were added to the residue and heated at 60C for nine hours. The solution was HPLC analyzed and measured 3.44g (8.98mmol) of α-L-aspartyl-D-valine-N-(S)-α-ethyl-p-chlorobenzyl amide. The yield was 80.2%.

The hydrolyzed solution was adjusted to pH6 with 25% NaOH and was concentrated under reduced pressure; the resulting slurry was ice cooled, and filtered at reduced pressure. The resulting α-L-aspartyl-D-valine-N-(S)--αethyl-p-chlorobenzyl amide crystals were rinsed with a small amount of water, ethyl acetate and chloroform, and then dried at reduced pressrue. The gained amount was 2.95g. The crystallization yield was 85.8%.

### Example 12

2.00g (19.4mmol) of D-α-aminobutyric acid, 2.25g (19.4mmol) of methyl acetoacetate and 3.52g (19.4mmol) of dicyclohexylamine were added to 17ml of methanol (Solution XII); Solution XII was refluxed while heating for three hours and concentrated at reduced pressure. 60ml of ethyl acetate and 0.20g (1.9mmol) of N-methylmorpholine were added to the resulting residue, and stared while cooling; 20ml of ethyl acetate solution containing 2.55ml (19.4mmol) of isobutyl chloroformate was added to the reaction solution while maintaining the temperature at -15C to -17C.
20 minutes later, 30ml of ethyl acetate solution containing 2.71g (16.2mmol) of (R)-α-metoxymethyl-p-hydroxybenzyl amine was added to the solution while maintaining the same temperature. The solution was warmed to the room temperature and filtered. 30ml of 1N HCl was added to the filtrate and stirred at the room temperature for ninety minutes. The aqueous layer was adjusted to pH11 with 1N NaOH, and was extracted with approximatey 70ml of ethyl acetate. The resulting ethyl acetate layer was dried over anhydrous magnesium sulfate; magnesium sulfate was filtered; the filtrate was concentrated under reduced pressures and measured 3.08g (12.2mmol) of D-α-aminobulyric acid-N-(R)-α-methoxymethyl-p-hydroxybenzylamide as clear light yellow syrup. The yield was 75.3%.

2.74g (14.9mmol) of L-aspartic acid β-methyl ester hydrochloride, 1.73g (14.9mmol) of methyl acetoacetate 5.41g (29.8mmol) of dicyclohexylamine were added to 25ml of metanol, and refluxed while heating for three hours, and then concentrated at reduced pressure. Ethyl acetate was added to the resulting residue and then the mixture was filtered. The filtrate was concentrated and it was obtained a syrup-like residue. 45ml of ethyl acetate and 0.75g (7.5mmol) of N-methylmorpholine were added to the residue, and the solution was cooled as described above; then ethyl acetate solution containing 1.96ml (14.9mmol) of isobutyl chloroformate was added. 20 minutes later, the solution made by dissolving 2.98g (11.8mmol) of said D-α-aminobulyric acid-N-(R)-α-metxymethyl-p-hydroxybenzyl amide into 30ml of ethyl acetate was added to the mixture. The solution was warmed to the room temperature, the reaction solution was concentrated under reduced pressure and the ethyl acetate distilled off. Some acetonitrile and 200ml of 2N HCl were added to the solution, and heated at 60C for six hours. The resulting mixture was HPLC analyzed and measured 3.38g (9.19mmol) of α-L-asparthyl-D-α-aminobulyric acid-N-(R)-α-methoxymethyl-p-hydroxybenzylamide. The yield was 77.9% This hydrolyzed solution was adjusted to pH6 with 25% NaOH and concentrated under reduced pressure; the resulting slurry was ice cooled under reduced presure. The resulting α-L-aspartyl-D-aminobutyric acid-N-(R)-α-methoxymethyl-p-hydroxybenzylamide were rinsed with a small amount of water, ethyl acetate and chloroform and dried at reduced pressure. The yield was 3.03g. The crystallization yield was 89.6%.

### Example 13

1.50g (8.17mmol) of L-aspartic acid β-methyl ester hydrochloride, 0.95g (8.2mmol) of methyl acetaoacetate and 5.78g (16.3mmol) of trioctylamine were added to 25ml of methanol (Solution XIII); Solution XIII was refluxed while heating for three hours and concentrated under reduced pressure. 30ml of ethyl acetate was added to the syrup-like residue and stirred while cooling to 10C, and 15ml of ethyl acetate solution containing 0.79ml (8.2mmol) of ethyl chloroformate was added to the mixture while maintaining the temperature at -11C to -12C. 20 minutes later, 20ml of ethyl acetate solution containing 1.84g (6.92mmol) of D-valine-N-(R)-α-methoxymethyl-p-hydroxybenzylamide was added while maintaining the temperature at -13C to -17C. The mixture was warmed to the room temperature, and 25ml of 1N HCl was added to the reaction solution and stirred at the room temperature for one hour. After the solution was separated into layers, the ethyl acetate layer was extracted twice with diluted hydrochloric acid. The aqueous layer was concentrated, and 15ml of 2N HCl was added to the resulting residue and heated at 60C for five hours. The hydrolyzed solution was HPLC analyzed and measured 1.92g (5.02mmol) of α-L-aspartyl-D-valine-N-(R)-α-methoxymethyl-p-hydroxybenzylamide. The yield was 72.5%.

The hydrolyzed solution was adjusted to pH6 with 25% NaOH, and the resulting slurry was stirred at 5C overnight, then filtered under reduced pressure. The resulting α-L-aspartyl-D-valine-N-(R)-α-methoxymethyl-p-hydroxybenzylamide crystals were rinsed with a small amount of water and dried at reduced pressure. The gained amount was 1.45g. The crystallization yield was 75.5%.

### EFFECTS OF THE INVENTION

The present invention relates to producing α-L-aspartyldipeptide amide derivative using economical protecting groups, activation agents and an easy process that does not yield β-peptide as a by-product. The invention can be used to produce the sweetener compounds, the α-L-aspartyl-D-amino acid-N-(S)-α-alkylbenzylamide of US Patent 5,286,509, the aspartyldipeptide derivative of Japanese Patent Application No.42818/1995, and the aspartyldipeptide amide derivatives of Japanese Patent Application No. 144844/1995.

## Claims

1. A process for producing an α-L-aspartyldipeptide amide derivative, which comprises mixing an L-aspartic acid derivative of formula (1) with alkyl chloroformate and with an amino acid amide derivative of formula (2), and hydrolyzing the reduction product under acidic conditions; 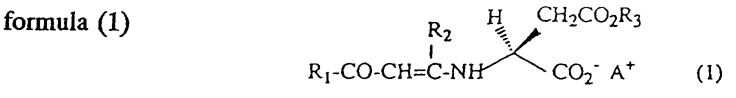 (Wherein R₁ is an alkyl or alkoxy group with 1 to 4 carbon atoms or an aromatic group with 6 to 10 carbon atoms; R₂ is an alkyl group with 1 to 4 carbon atoms or an aromatic group with 6 to 10 carbon atoms; R₃ is an alkyl group with 1 to 4 carbon atoms or a benzyl group; and A is a protonated tertiary amine, a dicyclohexylamine, or an alkaline metal,); 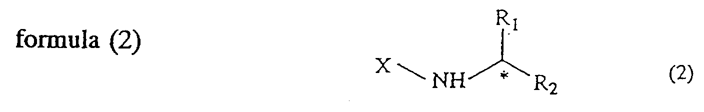 (Wherein R₁ is an alkyl group with 1 to 6 carbon atoms or an alkoxymethyl group with 2 to 7 carbon atoms; R₂ is a benzyl group, a cyclohexyl group, a cyclohexylmethyl group, a phenyl group, or a phenyl group with a substituent in its 2-, 3- or 4-position selected among F, Cl, Br I, a hydroxyl group, an alkoxy group with 1 to 6 carbon atoms, a cyano group, a nitro group, an acetyl group, an amino group, an acetylamino group, or a phenyl group with a methylenedioxy group, a trimethylene group, or a tetramethylene group in its 2, 3-or 3, 4- position, or a 2-, 3- or 4-pyridyl group, a 2- or 3-furyl group, or a 2- or 3-thienyl group; the configuration of C* is (S) or (RS) when R₁ is an alkyl group, and (R), (S), or (RS) when R₁ is an alkoxymethyl group,
X is a D-α-amino acid residue or a DL-α-amino acid residue selected from the group consisting of D-norleucine, D-leucine, D-isoleucine, D-alloisoleucine, D-threonine, D-O-methylthreonine, D-allothreonine, D-O-methylallothreonine, D-or DL-furylglycine, or cyclic or non-cyclic, α,α-dialkyl amino acid residue with 3 to 6 carbon atoms when R₁ is an alkyl group and R₂ is a benzyl group, a cyclohexyl group, a cyclohexylmethyl group, or a phenyl group;
a D-α-amino acid residue or a DL-α-amino acid residue selected from the group consisting of D-alanine, D-α-aminobutyric acid, D-norvaline, D-valine, D-norleucine, D-leucine, D-isoleucine, D-alloisoleucine, D-t-leucine, D-serine, D-O-methylserine, D-threonine,D-O-methylthreonine, D-allothreonine, D-O-methylallothreonine, D-phenylglycine,D- or DL-furylglycine, or a cyclic or non-cyclic α,α-dialkylamino acid residue with 3 to 6 carbon atoms when R₁ is an alkyl group and R₂ is a phenyl group with a substituent in its 2-, 3- or 4- position selected from the group consisting of F, Cl, Br, I, hydroxyl group, alkoxy groups with 1 to 6 carbon atoms, cyano group, nitro group, acetyl group, amino group, acetylamino group, or a phenyl group with methylenedioxy, trimethylene or tetramethylene groups in its 2, 3- or 3,4- position, or 2-, 3- or a 4-pyridyl group, a 2- or 3- furyl group or a 2- or 3-thienyl group ad when R₁ is an alkoxymethyl group, L-Asp and X are α-bonded.)

2. The process of claim 1, wherein the amino acid amide derivative is in an organic layer obtained by mixing the amino acid derivative of formula (3) with alkyl chloroformate, mixing the compound with the amine derivative of formula (4), contacting with acidic water to obtain an aqueous solution containing an amino acid amide derivative and extracting it with an organic solvent under alkaline condition. 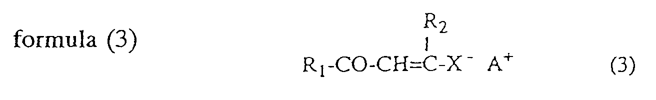 (Wherein R₁ is an alkyl or alkoxygroup with 1 to 4 carbon atoms, or an aromatic group with 6 to 10 carbon atoms; R₂ is an alkyl group with 1 to 4 carbon atoms or an aromatic group with 6 to 10 carbon atoms; X is a D-α-amino acid residue or a DL-α-amino acid residue selected from the group consisting of D-alanine, D-α-amino butyric acid, D-norvaline, D-valine, D-norleucine, D-leucine, D-isoleucine, D-alloisoleucine, D-t-leucine, D-serine, D-O-methylserine, D-threonine, D-O-methylthreonine, D-allothreonine, D-O-methylallothreonine or D-phenylglycine or D-or DL-furylglycine, or a cyclic or non-cyclic α,α-dialkyl amino acid residue with 3 to 6 carbon atoms; "A" represents protonated tertiary amine, dicyclohexylamine, or an alkaline metal.) 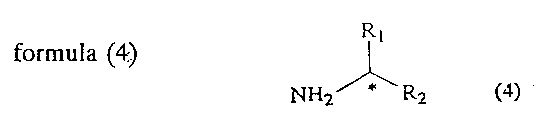 (Wherein R₁ is an alkyl group with 1 to 6 carbon atoms or an alkoxymethyl group with 2 to 7 carbon atoms; R₂ is a benzyl group, a cyclohexyl group, a cyclohexylmethyl group, a phenyl group, or a phenyl group with a substituent in its 2-, 3- or 4-position selected from the group consisting of F, Cl, Br, I, a hydroxyl group, an alkoxy group with 1 to 6 carbon atoms, a cyano group, a nitro group, an acetyl group, an amino group, an acetylamino group, or a phenyl group with a methylenedioxy group, a trimethylene group, a tetramethylene group in its 2, 3- or 3,4- position, or a 2-, 3- or 4-pyridyl group a 2- or 3-furyl group or a 2- or 3-thienyl group; the configuration of C* is (S) or (RS) when R₁ is an alkyl group; (R), (S), or (RS) when R₁ is an alkoxymethyl group.
